# EUROPEAN PATENT APPLICATION

(11) **EP 4 353 775 A1**
(43) Date of publication of application: **17.04.2024**
(21) Application number: 22804744.5
(22) Date of filing: 19.05.2022
(51) Int. Cl.: C08K 5/101, C08L 33/06

(54) **MONOMER COMPOSITION, METHACRYLIC RESIN COMPOSITION AND RESIN MOLDED BODY**

(30) Priority: 20.05.2021 JP 2021085291
(71) Applicant: Mitsubishi Chemical Corporation, Chiyoda-ku Tokyo 100-8251 (JP)
(72) Inventor: ITO, Hiroaki, Tokyo 100-8251 (JP); HIRANO, Yusuke, Tokyo 100-8251 (JP); TANIGUCHI, Issei, Tokyo 100-8251 (JP); ISOMURA, Manabu, Tokyo 100-8251 (JP); KANEMORI, Kouichi, Tokyo 100-8251 (JP)
(74) Representative: Ter Meer Steinmeister & Partner
(86) International application number: PCT/JP2022/020784
(87) International publication number: WO 2022/244835

(57) **Abstract**

Provided is a monomer composition containing methyl methacrylate, and at least one compound of methyl pyruvate and methyl 2-methylbutyrate.

## Description

### TECHNICAL FIELD

The present invention relates to a monomer composition, a methacrylic resin composition, and a resin molded body.

This application claims priority to Japanese Patent Application 2021-085291, filed on May 20, 2021, the contents of which are incorporated herein.

### BACKGROUND ART

Methacrylic resins have excellent transparency, heat resistance, and weather resistance, as well as well-balanced performance in terms of resin properties such as mechanical strength, thermal properties, and moldability. In particular, methacrylic resin plates made of methacrylic resin in plate shape are used for translucent components used in any of tanning beds, lighting equipment, skin therapy equipment, medical equipment, UV irradiation devices, equipment for growing animals and plants, skylights, HID lamps, and the like.

In the applications described above, when methacrylic resin plates are placed in an environment where they are exposed to UV, such as direct sunlight or a UV lamp, the methacrylic resin plates problematically have developed yellowing (yellow stain, yellowish color). Thus, there has been a demand for methacrylic resins that do not develop yellowing even when exposed to UV for long periods of time, i.e., methacrylic resins with excellent light stability.

As a technology for improving the light stability of methacrylic resins, Patent Document 1 discloses a methacrylic resin obtained by polymerizing a monomer such as methyl methacrylate in the presence of a light stabilizer, a hindered amine compound (HALS) having a specific structure.

### PRIOR ART DOCUMENTS

### PATENT DOCUMENTS

Patent Document 1: JP S55-139404 A

### SUMMARY

### PROBLEMS TO BE SOLVED

However, the methacrylic resin described in Patent Document 1 has shown increased light stability with the increase in the amount of HALS added during polymerization, but unfortunately rather been colored because HALS itself is colored.

Furthermore, unfortunately, the increase in the amount of HALS added has resulted in reduced polymerization efficiency and increased amounts of monomers remaining in the methacrylic resin, which in turn has reduced the light stability of the methacrylic resin.

In light of the circumstances described above, an object of the present invention is to provide a methacrylic resin composition with excellent light stability and reduced yellowing while maintaining the excellent heat resistance of a methacrylic resin; a resin molded body containing the methacrylic resin composition; and a monomer composition for obtaining the methacrylic resin composition can be provided.

### MEANS FOR SOLVING THE PROBLEMS

In order to solve the above problems, the present invention has the following characteristics. In other words, the gist of the present invention is as follows:
[1] A monomer composition comprising methyl methacrylate, and at least one compound of methyl pyruvate and methyl 2-methylbutyrate.
[2] A monomer composition comprising methyl methacrylate, and at least one compound of methyl pyruvate and methyl 2-methylbutyrate,
   wherein the total content of the at least one compound of methyl pyruvate and methyl 2-methylbutyrate is 5 ppm by mass or more relative to the total mass of the monomer composition.
[3] A monomer composition comprising methyl methacrylate, and at least one compound of methyl pyruvate and methyl 2-methylbutyrate,
   wherein the total content of the at least one compound of methyl pyruvate and methyl 2-methylbutyrate is 50 ppm by mass or more relative to the total mass of the monomer composition.
[4] A monomer composition comprising methyl methacrylate, and at least one compound of methyl pyruvate and methyl 2-methylbutyrate,
   wherein the total content of the at least one compound of methyl pyruvate and methyl 2-methylbutyrate is 100 ppm by mass or more relative to the total mass of the monomer composition.
[5] The monomer composition according to any one of [1] to [4], further comprising an acrylic ester.
[6] The monomer composition according to [5], wherein the acrylic ester is at least one compound selected from the group consisting of methyl acrylate, n-ethyl acrylate, and n-butyl acrylate.
[7] The monomer composition according to [5], wherein the acrylic ester is n-butyl acrylate.
[8] The monomer composition according to any one of [1] to [7], further comprising at least one compound of methyl isobutyrate and methyl propionate.
[9] A methacrylic resin composition comprising a radical polymerization product of a polymerizable composition (X2) comprising the monomer composition according to any one of [1] to [8].
[10] A methacrylic resin composition comprising methacrylic polymer (P), and at least one compound of methyl pyruvate and methyl 2-methylbutyrate.
[11] A methacrylic resin composition comprising methacrylic polymer (P), and at least one compound of methyl pyruvate and methyl 2-methylbutyrate,
   wherein the total content of the at least one compound of methyl pyruvate and methyl 2-methylbutyrate is 5 ppm by mass or more relative to the total mass of the methacrylic resin composition.
[12] A methacrylic resin composition comprising methacrylic polymer (P), and at least one compound of methyl pyruvate and methyl 2-methylbutyrate,
   wherein the total content of the at least one compound of methyl pyruvate and methyl 2-methylbutyrate is 50 ppm by mass or more relative to the total mass of the methacrylic resin composition.
[13] A methacrylic resin composition comprising methacrylic polymer (P), and at least one compound of methyl pyruvate and methyl 2-methylbutyrate,
   wherein the total content of the at least one compound of methyl pyruvate and methyl 2-methylbutyrate is 100 ppm by mass or more relative to the total mass of the methacrylic resin composition.
[14] The methacrylic resin composition according to any one of [10] to [13], wherein the methacrylic polymer (P) comprises 70 to 100% by mass of a repeating unit derived from methyl methacrylate, and 0 to 30% by mass of a repeating unit derived from an acrylic ester.
[15] The methacrylic resin composition according to any one of [10] to [13], wherein the methacrylic polymer (P) comprises 50 to 100% by mass of a repeating unit derived from methyl methacrylate, and 0 to 50% by mass of a repeating unit derived from styrene.
[16] A resin molded body comprising the methacrylic resin composition according to any one of [9] to [15].
[17] A method of producing a methacrylic resin composition, comprising a radical polymerization step of subjecting a polymerizable composition (X2) comprising the monomer composition according to any one of [1] to [8] to radical polymerization.

### ADVANTAGEOUS EFFECTS

According to the present invention, a methacrylic resin composition with excellent light stability and reduced yellowing while maintaining the excellent heat resistance of a methacrylic resin; a resin molded body containing the methacrylic resin composition; and a monomer composition for obtaining the methacrylic resin composition can be provided.

### DESCRIPTION OF EMBODIMENTS

As used herein, the term "(meth)acrylate" means at least one selected from "acrylate" and "methacrylate," and the term "(meth)acrylic" means at least one selected from "methacrylic" and "acrylic". "Methacrylic polymer" may include a repeating unit derived from an acrylic monomer in addition to a repeating unit derived from a methacrylic monomer.

As used herein, the term "monomer" means an unpolymerized compound, and the term "repeating unit" means a unit derived from the monomer, which is formed by polymerization of the monomer. The repeating unit may be a unit directly formed by a polymerization reaction, or may be a unit in which a part of the unit is converted into another structure by polymer treatment.

As used herein, the term "% by mass" indicates the content of a specific component contained in 100% by mass of the total amount.

Unless otherwise noted, any numerical value range indicated by the term "to" as used herein means any range including the numerical values described before and after the term "to" as the lower and upper limit values, respectively, and "A to B" means A or more and B or less.

As used herein, UV means light with a wavelength range from 295 nm to 430 nm, in other words, light mainly including light with a wavelength range of 380 nm or less.

### <1. Monomer Composition>

The monomer composition according to the first aspect of the present invention contains methyl methacrylate, and at least one compound of methyl pyruvate and methyl 2-methylbutyrate. Other components may also be contained without impairing the effects of the present invention.

### <1-1. Methyl Methacrylate>

The monomer composition according to the present aspect contains methyl methacrylate and can provide a methacrylic resin composition with good light stability and reduced yellowing.

The lower limit of the methyl methacrylate content relative to the total mass of the monomer composition according to the present aspect is not particularly limited, and is preferably 85% by mass or more, more preferably 90% by mass or more, still more preferably 95% by mass or more, and particularly preferably 97% by mass or more. The upper limit of the methyl methacrylate content is usually 99.9995% by mass or less, or may be 99.9950% by mass or less or 99.9900% by mass or less. Therefore, the methyl methacrylate content can be in the range of, for example, 85% by mass or more and 99.9995% by mass or less, 90% by mass or more and 99.9995% by mass or less, 95% by mass or more and 99.9950% by mass or less, or 97% by mass or more and 99.9900% by mass or less.

The total content of methyl methacrylate, methyl pyruvate, and methyl 2-methylbutyrate relative to the total mass of the monomer composition according to the present aspect is not particularly limited, and is usually 100% by mass or less.

### <1-2. Methyl Pyruvate and Methyl 2-Methylbutyrate>

The monomer composition according to the present aspect contains at least one compound of methyl pyruvate and methyl 2-methylbutyrate, and can provide a methacrylic resin composition with good light stability and reduced yellowing.

The lower limit of the total content of methyl pyruvate and methyl 2-methylbutyrate relative to the total mass of the monomer composition according to the present aspect is not particularly limited, and from the viewpoint that a methacrylic resin composition with better light stability can be provided, is usually 5 ppm by mass or more, preferably 50 ppm by mass or more, more preferably 100 ppm by mass or more, still more preferably 200 ppm by mass or more, and particularly preferably 300 ppm by mass or more.

The upper limit of the total content of methyl pyruvate and methyl 2-methylbutyrate relative to the total mass of the monomer composition according to the present aspect is not particularly limited, and from the viewpoint that, when the monomer composition is converted into a methacrylic resin composition, the heat resistance of the methacrylic resin is not impaired, is usually 50,000 ppm by mass or less, preferably 25,000 ppm by mass or less, more preferably 20,000 ppm by mass or less, still more preferably 15,000 ppm by mass or less, and particularly preferably 10,000 ppm by mass or less.

Any preferred upper and lower limit values described above can be combined.

Specifically, the total content of methyl pyruvate and methyl 2-methylbutyrate relative to the total mass of the monomer composition according to the present aspect is preferably 5 ppm by mass or more and 50,000 ppm by mass or less, more preferably 50 ppm by mass or more and 25,000 ppm by mass or less, still more preferably 100 ppm by mass or more and 20,000 ppm by mass or less, particularly preferably 200 ppm by mass or more and 15,000 ppm by mass or less, and most preferably 300 ppm by mass or more and 10,000 ppm by mass or less.

### <1-3. Other Monomers>

In addition to methyl methacrylate, the monomer composition according to the present aspect may contain a monomer other than methyl methacrylate. Examples of the monomer other than methyl methacrylate include the following monomers 1) to 16). One solely or two or more in any ratio and combination of the following monomers 1) to 16) can be used.
1) Methacrylic esters:
   for example, ethyl methacrylate, isopropyl methacrylate, n-butyl methacrylate, iso-butyl methacrylate, tert-butyl methacrylate, 2-ethylhexyl methacrylate, phenyl methacrylate, and benzyl methacrylate;
2) Acrylic esters:
   for example, methyl acrylate, ethyl acrylate, n-butyl acrylate, iso-butyl acrylate, tert-butyl acrylate, and 2-ethylhexyl acrylate;
3) Unsaturated carboxylic acids:
   for example, acrylic acid, methacrylic acid, maleic acid, and itaconic acid;
4) Unsaturated carboxylic anhydrides:
   for example, maleic anhydride, and itaconic anhydride;
5) Maleimides:
   for example, N-phenylmaleimide, and N-cyclohexylmaleimide;
6) Hydroxy group-containing vinyl monomers:
   for example, 2-hydroxyethyl acrylate, 2-hydroxyethyl methacrylate, and 2-hydroxypropyl methacrylate;
7) Vinyl esters:
   for example, vinyl acetate, and vinyl benzoate;
8) Vinyl chlorides, vinylidene chlorides, and derivatives thereof;
9) Nitrogen-containing vinyl monomers:
   such as methacrylamide, and acrylonitrile;
10) Epoxy group-containing monomers:
   for example, glycidyl acrylate, and glycidyl methacrylate;
11) Aromatic vinyl monomers:
   for example, styrene, and α-methylstyrene;
12) Alkane diol di(meth)acrylates:
   for example, ethylene glycol di(meth)acrylate, 1,2-propylene glycol di(meth)acrylate, 1,3-butyleneglycol di(meth)acrylate, and 1,6-hexanediol di(meth)acrylate;
13) Polyoxyalkylene glycol di(meth)acrylates:
   for example, diethylene glycol di(meth)acrylate, dipropylene glycol di(meth)acrylate, triethylene glycol (meth)acrylate, tetraethylene glycol di(meth)acrylate, polyethylene glycol di(meth)acrylate, and neopentyl glycol di(meth)acrylate;
14) Vinyl monomers having two or more ethylenically unsaturated bonds in their molecules:
   for example, divinylbenzene;
15) Unsaturated polyester prepolymers obtained from at least one polycarboxylic acid including ethylenically unsaturated polycarboxylic acid, and at least one diol; and
16) Vinyl ester prepolymers obtained by acrylic modification of an end of an epoxy group.

Among them, from the viewpoint of excellent balance of the transparency, heat resistance, and moldability of the methacrylic resin composition, the monomer is preferably at least one acrylic ester selected from the group consisting of methyl acrylate, ethyl acrylate, and n-butyl acrylate, and more preferably n-butyl acrylate. The acrylic ester content relative to the total mass of the monomer composition is preferably 0% by mass or more and 30% by mass or less.

### <1-4. Methyl Isobutyrate and Methyl Propionate>

The monomer composition according to the present aspect may further contain at least one compound of methyl isobutyrate and methyl propionate. The monomer composition contains the compound described above in addition to at least one compound of methyl pyruvate and methyl 2-methylbutyrate, and can provide a methacrylic resin composition with better light stability and further reduced yellowing.

The lower limit of the total content of methyl isobutyrate and methyl propionate relative to the total mass of the monomer composition according to the present aspect, from the viewpoint that a methacrylic resin composition with better light stability can be provided, is preferably 20 ppm by mass or more, more preferably 100 ppm by mass or more, still more preferably 200 ppm by mass or more, particularly preferably 500 ppm by mass or more, and most preferably 1,000 ppm by mass or more.

The upper limit of the total content of methyl isobutyrate and methyl propionate relative to the total mass of the monomer composition according to the present aspect is not particularly limited, and from the viewpoint that, when the monomer composition is converted into a methacrylic resin composition, the heat resistance of the methacrylic resin is not impaired, is usually 50,000 ppm by mass or less, preferably 25,000 ppm by mass or less, more preferably 10,000 ppm by mass or less, still more preferably 7,000 ppm by mass or less, and particularly preferably 5,000 ppm by mass or less.

Any preferred upper and lower limit values described above can be combined.

Specifically, the total content of methyl isobutyrate and methyl propionate can be in the range of 20 ppm by mass or more and 50,000 ppm by mass or less, 100 ppm by mass or more and 25,000 ppm by mass or less, 200 ppm by mass or more and 10,000 ppm by mass or less, 200 ppm by mass or more and 7,000 ppm by mass or less, 500 ppm by mass or more and 7,000 ppm by mass or less, or 1,000 ppm by mass or more and 5,000 ppm by mass or less. Among these, the total content of methyl isobutyrate and methyl propionate is more preferably 100 ppm by mass or more and 25,000 ppm by mass or less, and still more preferably 200 ppm by mass or more and 7,000 ppm by mass or less.

When the monomer composition contains at least one of methyl isobutyrate and methyl propionate, the total content of methyl isobutyrate, methyl propionate, methyl pyruvate, and methyl 2-methylbutyrate relative to the total mass of the monomer composition is preferably within the range of the total content of methyl pyruvate and methyl 2-methylbutyrate as described above.

### <1-5. Additives>

It is considered that in the present aspect, methyl pyruvate and methyl 2-methylbutyrate develop excellent light stability by a different mechanism of action from those of commonly known UV absorbers and radical scavengers (HALS). Thus, at least one compound of methyl pyruvate and methyl 2-methylbutyrate can also be used in combination with additives such as UV absorber and HALS. The monomer composition contains at least one compound of methyl pyruvate and methyl 2-methylbutyrate, and the additives, and can provide a methacrylic resin composition and a resin molded body with increased light stability at a lower cost.

Examples of the additives include known additives such as mold release agents, lubricants, plasticizers, antioxidants, antistatic agents, light stabilizers other than methyl pyruvate or methyl 2-methylbutyrate, ultraviolet absorbers, flame retardants, flame retardant promoters, polymerization inhibitors, fillers, pigments, dyes, silane coupling agents, leveling agents, antifoaming agents, and fluorescent agents. One solely or two or more in any combination of the additives can be used.

The monomer composition according to the present aspect may also contain a compound that is inevitably mixed into methyl methacrylate, such as methacrolein and methanol.

### <2. Polymerizable Composition (X2)>

The polymerizable composition (X2) according to the second aspect of the present invention is one embodiment of the raw material to obtain the methacrylic resin composition according to the third aspect of the present invention described later. The polymerizable composition (X2) according to the present aspect is, for example, a polymerizable composition (X2-1) containing a raw material composition (X1) as described later, at least one compound of methyl pyruvate and methyl 2-methylbutyrate, and a known radical polymerization initiator; or a polymerizable composition (X2-2) containing a monomer composition according to the first aspect of the present invention and a known radical polymerization initiator.

### <2-1. Raw Material Composition (X1)>

The raw material composition (X1) is a component of the polymerizable composition (X2-1), and is also a raw material component of the methacrylic polymer (P) contained in the methacrylic resin composition according to the third aspect of the present invention. The description below will be mainly on a raw material composition (X1) used in manufacturing of a methacrylic polymer (P1) as the methacrylic polymer (P), containing a methyl methacrylate (hereinafter also referred to as "MMA")-derived repeating unit (hereinafter also referred to as "MMA unit") and an acrylic ester-derived repeating unit (hereinafter also referred to as "acrylic ester unit"). By changing the acrylic ester to styrene, the raw material composition can also be applied in manufacturing of a methacrylic polymer (P2) containing an MMA unit and a styrene-derived repeating unit (hereinafter also referred to as "styrene unit"). In this case, as the styrene content, the styrene unit content as described in <3-1. Methacrylic Polymer (P)> can be applied.

Examples of the raw material composition (X1) include a composition only containing MMA, and a composition containing the MMA and an acrylic ester. As the acrylic ester, the same monomer as the acrylic esters as described in <1-3. Other Monomers> or as the acrylic esters as described in <3-1. Methacrylic Polymer (P)> can be used.

The raw material composition (X1) contains MMA and an acrylic ester, resulting in improved light stability of the methacrylic resin composition, making it possible to prevent the occurrence of yellowing when the resin molded body containing the methacrylic resin composition is exposed to UV for long periods of time and the reduction of light stability.

The MMA content in the raw material composition (X1) is not particularly limited, and from the viewpoint that the light stability of the methacrylic resin composition can be improved, the same content as the MMA unit content in the methacrylic polymer (P1) or methacrylic polymer (P2) as described in <3-1. Methacrylic Polymer (P)> can be suitably used. However, the phrases "relative to the total mass of the methacrylic polymer (P1)" and "relative to the total mass of the methacrylic polymer (P2)" in <3-1. Methacrylic Polymer (P)> shall be read as "relative to the total mass of the raw material composition (X1)."

The acrylic ester (M2) content in the raw material composition (X1) is not particularly limited, and from the viewpoint that the light stability of the methacrylic resin composition can be improved, the same content as the acrylic ester unit content contained in the methacrylic polymer (P1) or methacrylic polymer (P2) as described in <3-1. Methacrylic Polymer (P)> can be suitably used. However, the phrases "relative to the total mass of the methacrylic polymer (P1)" and "relative to the total mass of the methacrylic polymer (P2)" in <3-1. Methacrylic Polymer (P)> shall be read as "relative to the total mass of the raw material composition (X1)."

With respect to the type of the acrylic ester, from the viewpoint of excellent light stability of the methacrylic resin composition, the same compound as the acrylic esters as described in <1-3. Other Monomers> or as the acrylic esters as described in <3-1. Methacrylic Polymer (P)> can be applied.

The raw material composition (X1) can previously contain a polymer containing an MMA unit. Specifically, the raw material composition (X1) can previously contain a polymer (a) as described later. When the raw material composition (X1) contains the polymer (a), the polymerizable composition (X2-1) is viscous liquid (called "syrup"), thus making it possible to shorten the polymerization time and improve the productivity.

The method to obtain the syrup described above may be, for example, a method in which a polymer is dissolved in the raw material composition (X1), or a method in which a known radical polymerization initiator is added to the raw material composition (X1), followed by allowing a part of the mixture to be polymerized.

When the polymerizable composition (X2-1) is a syrup, the polymerizable composition (X2-1) may be a composition containing a polymer (a) and a monomer composition (m) as described below:
Polymer (a): a polymer containing 70.0% by mass or more of an MMA unit and 30.0% by mass or less of the acrylic ester unit, or a polymer containing 50.0% by mass or more of an MMA unit and 50.0% by mass or less of the styrene unit, or a polymer containing 100% by mass of an MMA unit, relative to the total mass of the polymer (a);
Monomer composition (m): a monomer composition containing 70.0% by mass or more of MMA and 30.0% by mass or less of an acrylic ester, or a monomer composition containing 50.0% by mass or more of MMA and 50.0% by mass or less of styrene, or a monomer composition containing 100% by mass of MMA, relative to the total mass of the monomer composition (m).

The raw material composition (X1) content (unit: % by mass) contained in the polymerizable composition (X2-1) is not particularly limited, and can be in the range of 97.5% by mass or more and 99.99% by mass or less relative to the total mass of the polymerizable composition (X2-1).

### <2-2. Monomer Composition>

The monomer composition that is a component of the polymerizable composition (X2-2) is the monomer composition according to the first aspect of the present invention, and is a composition containing a raw material component for a methacrylic polymer (P) contained in the methacrylic resin composition according to the third aspect of the present invention.

The content of the monomer composition according to the first aspect of the present invention with respect to the total mass of the polymerizable composition (X2-2) is 60% by mass or more and less than 100% by mass.

The polymerizable composition (X2-2) may also contain other monomers (also simply referred to as "other monomers") copolymerizable with the monomer in the monomer composition. When the polymerizable composition (X2-2) contains other monomers, the other monomers content is more than 0% by mass and less than 40% by mass relative to the total mass of the polymerizable composition (X2-2).

The other monomers include monomers 1) to 16) cited in <1-3. Other Monomers> as described above.

One solely or two or more in any ratio and combination of the monomers 1) to 16) described above can be used.

Among the monomers 1) to 16) described above, from the viewpoint that a methacrylic resin composition with excellent balance between heat resistance and transparency, a monomer selected from ethylene glycol dimethacrylate and neopentylglycol dimethacrylate is preferred.

### <2-3. Radical Polymerization Initiator>

Examples of the radical polymerization initiator include known azo compounds such as 2,2'-azobis(isobutyronitrile) and 2,2'-azobis(2,4-dimethylvaleronitrile); and known organic peroxides such as benzoyl peroxide and lauroyl peroxide. One solely or two or more in any ratio and combination thereof can be used. Known polymerization promoters such as amines and mercaptans can also be used, as necessary, in combination with the radical polymerization initiator.

The radical polymerization initiator content in the polymerizable composition (X2) is not particularly limited, and can be determined as appropriate according to known techniques by those skilled in the art. Specifically, the radical polymerization agent content relative to 100 parts by mass of the total mass of the polymerizable composition (X2) may be 0.005 parts by mass or more and 5 parts by mass or less, or 0.01 parts by mass or more and 1.0 part by mass or less.

### <2-4. Additives>

The polymerizable composition (X2) may contain additives selected from mold release agents, heat stabilizing agents, lubricants, plasticizers, antioxidants, antistatic agents, light stabilizers other than methyl pyruvate and methyl 2-methylbutyrate, ultraviolet absorbers, flame retardants, flame retardant promoters, polymerization inhibitors, fillers, pigments, dyes, silane coupling agents, leveling agents, antifoaming agents, fluorescent agents, chain transfer agents, and the like, as necessary.

### <3. Methacrylic Resin Composition>

The methacrylic resin composition according to the third aspect of the present invention (hereinafter also referred to as "methacrylic resin composition") is a methacrylic resin composition containing at least methacrylic polymer(P), and at least one compound of methyl pyruvate and methyl 2-methylbutyrate. The methacrylic resin composition according to the present aspect may be a composition obtained by radical polymerization of the polymerizable composition (X2) according to the second aspect of the present invention.

The methacrylic resin composition according to the present aspect contains a methacrylic polymer (P), and can provide a resin molded body with good transparency.

The methacrylic resin composition contains at least one compound of methyl pyruvate and methyl 2-methylbutyrate, and can provide a resin molded body with reduced occurrence of yellowing even when the resin molded body is exposed to UV for long periods of time and reduced degradation of light stability.

The state of the methacrylic resin composition is not particularly restricted, and is usually solid.

The methacrylic polymer (P) content relative to the total mass of the methacrylic resin composition is not particularly limited, and from the viewpoint of good heat resistance, is usually 95% by mass or more, preferably 97.5% by mass or more, more preferably 98% by mass or more, and still more preferably 99.0% by mass or more. From the viewpoint of excellent light stability, the content is usually 99.9995% by mass or less, and is preferably 99.9950% by mass or less, 99.99% by mass or less, 99.9850% by mass or less, 99.98% by mass or less, 99.97% by mass or less, 99.95% by mass or less, or 99.90% by mass or less. Any upper and lower limit values described above can be combined. For example, a preferred methacrylic polymer (P) content can be in the range of 95% by mass or more and 99.9995% by mass or less, 95% by mass or more and 99.9950% by mass or less, 97.5% by mass or more and 99.99% by mass or less, 98% by mass or more and 99.9850% by mass or less, 99.0% by mass or more and 99.98% by mass or less, 99.0% by mass or more and 99.97% by mass or less, 99.0% by mass or more and 99.95% by mass or less, or 99.0% by mass or more and 99.90% by mass or less. It is noted that when the methacrylic resin composition contains two or more methacrylic polymers (P), the content described above is the total content of the two or more methacrylic polymers (P).

The total content of the at least one compound of methyl pyruvate and methyl 2-methylbutyrate relative to the total mass of the methacrylic resin composition is not particularly limited. The total content of the at least one compound of methyl pyruvate and methyl 2-methylbutyrate relative to the total mass of the methacrylic resin composition, from the viewpoint of excellent light stability, is usually 5 ppm by mass or more, preferably 50 ppm by mass or more, more preferably 100 ppm by mass or more, still more preferably 150 ppm by mass or more, particularly preferably 200 ppm by mass or more, and most preferably 300 ppm by mass or more.

The upper limit of the total content of at least one compound of methyl pyruvate and methyl 2-methylbutyrate contained in the methacrylic resin composition according to the present aspect is not particularly limited, and from the viewpoint of good heat resistance of the resin molded body, is usually 50,000 ppm by mass or less, preferably 25,000 ppm by mass or less, more preferably 20,000 ppm by mass or less, still more preferably 15,000 ppm by mass or less, and particularly preferably 10,000 ppm by mass or less.

Any upper and lower limit values described above can be combined. For example, a preferred total content of at least one compound of methyl pyruvate and methyl 2-methylbutyrate can be in the range of 5 ppm by mass or more and 50,000 ppm by mass or less, 50 ppm by mass or more and 25,000 ppm by mass or less, 100 ppm by mass or more and 20,000 ppm by mass or less, 150 ppm by mass or more and 15,000 ppm by mass or less, 200 ppm by mass or more and 15,000 ppm by mass or less, or 300 ppm by mass or more and 10,000 ppm by mass or less. Among them, the total content of at least one compound of methyl pyruvate and methyl 2-methylbutyrate is more preferably 50 ppm by mass or more and 25,000 ppm by mass or less, and still more preferably 150 ppm by mass or more and 15,000 ppm by mass or less.

It is considered that in the present aspect, at least one compound of methyl pyruvate and methyl 2-methylbutyrate develop excellent light stability by a different mechanism of action from those of commonly known UV absorbers and radical scavengers (HALS). Thus, at least one compound of methyl pyruvate and methyl 2-methylbutyrate can also be used in combination with additives such as UV absorber and HALS. The use of at least one compound of methyl pyruvate and methyl 2-methylbutyrate, and the additives in combination can provide a methacrylic resin composition and a resin molded body with increased light stability at a lower cost.

The methacrylic resin composition may contain other components than methacrylic polymer (P) and at least one compound of methyl pyruvate and methyl 2-methylbutyrate within such a range that the effects of the present invention can be obtained. For example, the methacrylic resin composition may contain additives selected from mold release agents, heat stabilizers, antioxidants, ultraviolet absorbers, light stabilizers other than methyl pyruvate and methyl 2-methylbutyrate, and the like.

### <3-1. Methacrylic Polymer (P)>

The methacrylic polymer (P) is one of the components contained in the methacrylic resin composition according to the present aspect.

The methacrylic resin composition contains a methacrylic polymer (P), and can have improved transparency as well as reduced thermal- or photo-decomposition resulting in good heat moldability, heat resistance, and mechanical strength. Furthermore, the synergistic effect of the inherent heat resistance of methacrylic polymer (P) and at least one compound of methyl pyruvate and methyl 2-methylbutyrate prevents the occurrence of yellowing during exposure to UV for long periods of time, making it possible to obtain a methacrylic resin molded body with high light stability and maintained heat resistance.

The methacrylic polymer (P) is preferably a copolymer containing an MMA unit and an acrylic ester unit (hereinafter also referred to as methacrylic polymer (P1)), or a copolymer containing an MMA unit and a styrene unit (hereinafter also referred to as methacrylic polymer (P2)). The sequences of the copolymers are not particularly restricted, and the copolymer may be, for example, a random copolymer, a block copolymer, or an alternating copolymer, and preferably a random copolymer.

The acrylic ester-derived repeating unit is an acrylic ester-derived repeating unit having a C₁₋₆ alkyl group on its side chain. The monomer constituting this unit is not particularly limited as long as it is a monomer copolymerizable with MMA. Examples include acrylic esters, such as methyl acrylate, ethyl acrylate, propyl acrylate, n-butyl acrylate, and t-butyl acrylate. One solely or two or more in any ratio and combination thereof may be used. Among these monomers, from the viewpoint of reducing the occurrence of yellowing when the resin molded body containing the methacrylic resin composition is exposed to UV for long periods of time and ensuring high light stability, the monomer is preferably at least one acrylic ester selected from the group consisting of methyl acrylate, n-ethyl acrylate, and n-butyl acrylate, and more preferably n-butyl acrylate.

The MMA unit content in the methacrylic polymer (P1) is not particularly limited. From the viewpoint of good heat resistance, the MMA unit content relative to the total mass of the methacrylic polymer (P1) is preferably 70.0% by mass or more, still more preferably 80.0% by mass or more, still more preferably 90.0% by mass or more, and usually 100% by mass or less.

The acrylic ester unit content in the methacrylic polymer (P1) is not particularly limited, and from the viewpoint of good heat resistance and light stability, is preferably 30% by mass or less, more preferably 20% by mass or less, still more preferably 10% by mass or less, and usually 0% by mass or more. It is noted that when the methacrylic polymer (P1) contains two or more acrylic ester units, the content described above is the total content of the two or more acrylic ester units.

The MMA unit content in the methacrylic polymer (P2) is not particularly limited, and from the viewpoint of good heat resistance, is preferably 50.0% by mass or more, more preferably 60.0% by mass or more, still more preferably 70.0% by mass or more, and usually 100% by mass or less, relative to the total mass of the methacrylic polymer (P2).

The styrene unit content in the methacrylic polymer (P2) is not particularly limited, and from the viewpoint of good transparency, is preferably 50% by mass or less, more preferably 40% by mass or less, still more preferably 30% by mass or less, and usually 0% by mass or more.

The methacrylic polymer (P) in the present aspect can further contain, within such a range that the effects of the present invention can be obtained, a structural unit derived from a polyfunctional monomer containing two or more radical polymerizable functional groups in one molecule (hereinafter referred to as "polyfunctional monomer unit").

The radical polymerizable functional group, as used herein, may be any radical polymerizable group having a carbon-carbon double bond, and specific examples thereof include a vinyl group, an allyl group, a (meth)acryloyl group, and a (meth)acryloyloxy group. Especially, a (meth)acryloyl group is preferred from the viewpoints of excellent storage stability of the compound having the radical polymerizable functional group and of ease of controlling the polymerizability of the compound. It is noted that "(meth)acryloyl" represents one or both of "acryloyl" and "methacryloyl." In a monomer having two radical polymerizable functional groups, the radical polymerizable functional groups may be the same or different.

Inclusion of a polyfunctional monomer unit in the methacrylic polymer (P) can improve the solvent resistance, chemical resistance, or the like.

Examples of the polyfunctional monomer include, but not particularly limited to, allyl methacrylate, allyl acrylate, ethylene glycol di(meth)acrylate, ethylene glycol tri(meth)acrylate, neopentyl glycol di(meth)acrylate, and trimethylolpropane tri(meth)acrylate. One solely or two or more in any ratio and combination thereof may be used. Among them, from the viewpoint of better solvent resistance and chemical resistance, the polyfunctional monomer is more preferably selected from ethylene glycol di(meth)acrylate and neopentylglycol di(meth)acrylate, and still more preferably is ethylene glycol di(meth)acrylate.

In the methacrylic resin composition according to the present aspect, the weight average molecular weight (Mw) of the methacrylic polymer (P), as measured by gel permeation chromatography (GPC), is not particularly limited. The weight average molecular weight (Mw) can be set as appropriate according to the use of the resin molded body or the like. For example, the weight average molecular weight may be 10,000 or more, 100,000 or more, or 150,000 or more, and may be 1,000,000 or less, 2,000,000 or less, or 4,000,000 or less.

The weight average molecular weight is defined as a value measured by gel permeation chromatography using standard polystyrene as a standard sample. The solvent resistance and chemical resistance can be increased by increasing the weight average molecular weight as appropriate.

The weight average molecular weight (Mw) of the methacrylic polymer (P) can be controlled by adjusting the polymerization temperature, the polymerization time, the amount of the polymerization initiator added, or the type or amount of the chain transfer agent added.

### <3-2. Methyl Pyruvate and Methyl 2-methylbutyrate>

At least one compound of methyl pyruvate and methyl 2-methylbutyrate is one of the components contained in the methacrylic resin composition according to the present aspect. The methacrylic resin composition contains at least one compound of methyl pyruvate and methyl 2-methylbutyrate, and can reduce the occurrence of yellowing when exposed to UV for long periods of time. Furthermore, methyl isobutyrate is less expensive than conventional ultraviolet absorbers and the like, and can also reduce the degradation of light stability.

### <3-3. Methyl Isobutyrate and Methyl Propionate>

The methacrylic resin composition according to the present aspect may further contain at least one compound of methyl isobutyrate and methyl propionate.

The lower limit of the total content of methyl isobutyrate and methyl propionate relative to the total mass of the methacrylic resin composition according to the present aspect, from the viewpoint that a methacrylic resin composition with better light stability can be provided, is preferably 20 ppm by mass or more, more preferably 100 ppm by mass or more, still more preferably 200 ppm by mass or more, particularly preferably 500 ppm by mass or more, and most preferably 1,000 ppm by mass or more.

The upper limit of the total content of methyl isobutyrate and methyl propionate relative to the total mass of the methacrylic resin composition according to the present aspect is not particularly limited, and from the viewpoint that the heat resistance of the methacrylic resin is not impaired, is usually 50,000 ppm by mass or less, preferably 25,000 ppm by mass or less, more preferably 10,000 ppm by mass or less, still more preferably 7,000 ppm by mass or less, and particularly preferably 5,000 ppm by mass or less.

Any preferred upper and lower limit values described above can be combined.

Specifically, the total content of methyl isobutyrate and methyl propionate can be in the range of 20 ppm by mass or more and 50,000 ppm by mass or less, 100 ppm by mass or more and 25,000 ppm by mass or less, 200 ppm by mass or more and 10,000 ppm by mass or less, 200 ppm by mass or more and 7,000 ppm by mass or less, 500 ppm by mass or more and 7,000 ppm by mass or less, or 1,000 ppm by mass or more and 5,000 ppm by mass or less. Among these, the total content of methyl isobutyrate and methyl propionate is more preferably 100 ppm by mass or more and 25,000 ppm by mass or less, and still more preferably 200 ppm by mass or more and 7,000 ppm by mass or less.

When the methacrylic resin composition contains at least one compound of methyl isobutyrate and methyl propionate, the total content of methyl isobutyrate, methyl propionate, methyl pyruvate, and methyl 2-methylbutyrate relative to the total mass of the methacrylic resin composition is preferably within the range of the total content of methyl pyruvate and methyl 2-methylbutyrate as described above.

### <3-4. Characteristics of Methacrylic Resin Composition>

The methacrylic resin composition according to the present aspect contains the methacrylic polymer (P), and at least one compound of methyl pyruvate and methyl 2-methylbutyrate, and thus has excellent light stability.

Specifically, when a test piece composed of the methacrylic resin composition (square shape with 50 mm of length × 50 mm of width, and thickness of 3 mm) is subjected to a UV exposure test as shown below, the yellowness index (YI) of the test piece obtained between before and 200 hours after the start of the UV exposure test, as measured in accordance with ASTM D 1925, is 7.1 or less, preferably 6.0 or less, more preferably 4.5 or less, and still more preferably 4.0 or less. In addition, suitably with respect to the test piece, the light transmittance at a wavelength of 295 nm is 15.0% or more, or the light transmittance at a wavelength of 315 nm is 35.0% or more. More suitably with respect to the test piece, the light transmittance at a wavelength of 295 nm is 15.0% or more, and the light transmittance at a wavelength of 315 nm is 35.0% or more. The light transmittance means the total light transmittance (Tt) of the test piece in the direction of thickness, measured in accordance with JIS K 7361-1:1997 using a haze meter (e.g., "NDH4000" manufactured by Nippon Denshoku Industries Co., Ltd.).

### (UV Exposure Test Method)

In the evaluation chamber of METAL WEATHER ultra-accelerating light stability tester (e.g., "KU-R5CI-A" manufactured by Daipla Wintes Co., Ltd.) equipped with a metal-halide lamp (e.g., "MW-60W" manufactured by Daipla Wintes Co., Ltd.) and a light-cutting filter (e.g., "KF-1" manufactured by Daipla Wintes Co., Ltd.), a test piece composed of the methacrylic resin composition (square shape with 50 mm of length × 50 mm of width, and thickness of 5 mm) is placed, and then irradiated with ultraviolet light (radiation intensity: 80 mW/cm²) from the metal-halide lamp at a temperature of 63°C and a humidity of 50% (RH).

### <4. Resin Molded Body>

The resin molded body according to the fourth aspect of the present invention (also simply referred to as "resin molded body") is a resin molded body containing the methacrylic resin composition according to the third aspect of the present invention. By molding the methacrylic resin composition, a resin molded body having excellent light stability can be obtained. The resin molded body, as used herein, is not particularly limited as long as it is a molded body containing the methacrylic resin composition described above, and a molded body only containing the methacrylic resin composition substantially corresponds to both methacrylic resin composition and resin molded body.

The shape of the resin molded body may be, for example, a plate resin molded body (resin plate) or a sheet resin molded body (resin sheet). The thickness of the resin molded body can be adjusted to any thickness, from thick plate to thin film, as necessary. For example, the thickness can be 1 mm or more and 30 mm or less.

The resin molded body contains the methacrylic resin composition as described above, and thus has excellent light stability.

In other words, a test piece of the resin molded body (square shape with 50 mm of length × 50 mm of width, and thickness of 3 mm) shows such excellent light stability that the yellowness index (YI) obtained between before and 200 hours after the start of the UV exposure test as described above, as measured in accordance with ASTM D 1925, is 7.1 or less, preferably 6.0 or less, more preferably 4.5 or less, and still more preferably 4.0 or less. In addition, the test piece suitably shows such high light stability that the light transmittance at a wavelength of 295 nm is 15.0% or more, or the light transmittance at a wavelength of 315 nm is 35.0% or more. More suitably with respect to the test piece, the light transmittance at a wavelength of 295 nm is 15.0% or more, and the light transmittance at a wavelength of 315 nm is 35.0% or more. The light transmittance means the total light transmittance (Tt) of the test piece in the direction of thickness, measured in accordance with JIS K 7361-1:1997 using a haze meter (e.g., "NDH4000" manufactured by Nippon Denshoku Industries Co., Ltd.).

### <5. Method of Producing Methacrylic Resin Composition or Resin Molded Body>

The method of producing a methacrylic resin composition, or a resin molded body containing the resin composition (hereinafter, the methacrylic resin composition and the resin molded body are also collectively referred to as "resin composition and the like") is not particularly limited. Specific examples of the method of producing the resin composition and the like include a method including a radical polymerization step of subjecting the polymerizable composition (X2) according to the second aspect of the present invention, preferably a polymerizable composition (X2-2) containing the monomer composition according to the first aspect of the present invention to radical polymerization. The radical polymerization step may include a syrup preparation step of polymerizing a part of the polymerizable composition (X2) to prepare a syrup, and a polymerization step of polymerizing a polymerizable component in the syrup. The phrase "polymerizing a part of the polymerizable composition (X2)" in the syrup preparation step means such polymerization that the methacrylic polymer content in the obtained syrup is 10% by mass or more and 80% by mass or less, preferably 10% by mass or more and 60% by mass or less, and more preferably 10% by mass or more and 40% by mass or less.

The polymerization temperature during the polymerization of the polymerizable composition (X2) is not particularly limited, and can be determined as appropriate according to known techniques by those skilled in the art. Usually, the polymerization temperature is set as appropriate according to the type of the radical polymerization initiator used, preferably in the range of 40°C or higher and 180°C or lower, more preferably 50°C or higher and 150°C or lower. The polymerizable composition (X2) may be polymerized under multi-step temperature conditions as necessary. The polymerization time is preferably determined as appropriate according to the progress of the polymerization and curing.

Examples of the method of polymerizing the polymerizable composition (X2) include a bulk polymerization method, a suspension polymerization method, an emulsion polymerization method, and a dispersion polymerization. Among them, a bulk polymerization method is preferred in view of productivity.

Specific examples of the method of producing a resin composition and the like include a method in which a bulk polymerization method using a known cast polymerization method such as a cell casting method or a serial casting method is used to obtain a resin composition and the like, and a method in which a composition produced by a bulk polymerization method is molded by an extrusion molding method, an injection molding method, or the like to obtain a resin composition and the like. From the viewpoint of being able to further improve the heat resistance of the methacrylic resin composition by increasing the molecular weight and introducing a cross-linked structure, it is more preferable to use a method using a cast polymerization method.

In order to obtain a resin composition and the like with a plate shape, the cast polymerization method may be, for example, a cell casting method in which using as a mold a space formed by two glass plates or metal plates (SUS plates) facing one another and gaskets such as soft resin tubes placed around the edges, a polymerizable composition (X2) or a syrup obtained by polymerizing a part of the polymerizable composition (X2) is injected into the mold, and subjected to a heat polymerization process to complete polymerization, and then the resin composition and the like are removed from the mold. Alternatively, the cast polymerization method may be a serial casting method in which using as a mold a space formed by two stainless endless belts which face one another at a predetermined distance and run in the same direction at the same speed, and gaskets such as soft resin tubes placed on both sides thereof, a polymerizable composition (X2) or a syrup obtained by polymerizing a part of the polymerizable composition (X2) is serially injected from one end of the endless belt into the mold, and subjected to a heat polymerization process to complete polymerization, and then the resin composition and the like are serially removed from the other end of the endless belt.

A resin composition and the like having a desired thickness can be obtained by adjusting as appropriate the spacing of the void in a mold by changing the thickness (diameter) of the gaskets. The thickness of the plate resin composition and the like is usually set in a range from 1 mm to 30 mm.

### <6. Use>

The use of the methacrylic resin composition and the resin molded body ("resin composition and the like") as described above is not particularly limited, and they are preferably used as translucent components used in any of tanning beds, lighting equipment, skin therapy equipment, medical equipment, UV irradiation devices, equipment for growing animals and plants, skylights, HID lamps, and the like, particularly as transparent components. More specifically, the resin composition and the like are preferably used as lighting components, which are components for the purpose of harvesting light, used in any of tanning beds, skylights, and the like, or as translucent components, which are components for the purpose of allowing light pass through themselves, used in any of lighting equipment, skin therapy equipment, medical equipment, UV irradiation devices, equipment for growing animals and plants, HID lamps, and the like.

### <7. Functions and Effects>

The monomer composition according to the first aspect of the present invention contains at least one compound of methyl pyruvate and methyl 2-methylbutyrate. A methacrylic resin composition obtained by radical polymerization of a polymerizable composition (X2) containing the monomer composition has excellent light stability and reduced yellowing while maintaining excellent heat resistance.

The reason why a methacrylic resin composition having excellent light stability and reduced yellowing while maintaining excellent heat resistance is obtained because the monomer composition according to the first aspect of the present invention contains at least one compound of methyl pyruvate and methyl 2-methylbutyrate is inferred as follows.

A polymer containing a methyl methacrylate-based unit (methacrylic polymer) produces radical species via cleavage of its main chain or side chains due to light. Usually, this produced radical species in turn cause yellowing and reduction of mechanical strength due to reduced molecular weight in the methacrylic resin.

However, it is considered that since at least one compound of methyl pyruvate and methyl 2-methylbutyrate contained in the monomer composition according to the first aspect of the present invention is remained in the methacrylic resin composition, the at least one compound of methyl pyruvate and methyl 2-methylbutyrate functions as a radical scavenger. This is considered to allow the methacrylic resin composition to exhibit excellent heat resistance and good light stability.

### EXAMPLES

The characteristics of the present invention will now be described in more detail with reference to Examples and Comparative Examples The materials, amounts used, ratios, treatment contents, treatment procedures, and the like shown in the following examples can be changed as appropriate without departing from the spirit and scope of the present invention. Thus, the scope of the present invention should not be interpreted as limited by the specific examples shown below. In the following description, the term "part" represents "part by mass."

The abbreviations and names of the compounds used in Examples and Comparative Examples are as follows:
- MMA: methyl methacrylate (Mitsubishi Chemical Corporation)
- BA: n-butyl acrylate (Mitsubishi Chemical Corporation)
- methyl isobutyrate (Tokyo Chemical Industry Co., Ltd.)
- methyl propionate (Tokyo Chemical Industry Co., Ltd.)
- methyl pyruvate (Tokyo Chemical Industry Co., Ltd.)
- methyl 2-methylbutyrate (Tokyo Chemical Industry Co., Ltd.)
- LA-57: 1,2,3,4-butanetetracarboxylic acid tetrakis(2,2,6,6-tetramethyl-4-piperidinyl) ester (ADEKA Corporation).

MMA (Mitsubishi Chemical Corporation) contained methyl isobutyrate at a concentration of 260 ppm by mass, methyl propionate at a concentration of 8 ppm by mass, methyl pyruvate at a concentration of 8 ppm by mass, and methyl 2-methylbutyrate at a concentration of 8 ppm by mass, relative to the total mass of MMA.

### [Methods for Measurement and Evaluation]

### <Method of Measuring Amount of Target Substance Remaining in Methacrylic Resin>

### (1) Procedure for Preparing Sample and Test Solution

Resin molded bodies obtained in Examples and Comparative Examples were finely crushed, and 0.2 g of the crushed resins were dissolved in 10 mL of acetone for pesticide residue test (hereinafter simply referred to as "acetone"). After the resins were dissolved, 1 mL of an internal standard solution was added with a whole pipet. As the internal standard solution, a 0.1 vol% methyl salicylate/acetone solution was used. A standard sample for the target was diluted with acetone to prepare three test solutions having different concentrations. Gas chromatography-mass spectrometry (GC/MS) measurement was performed as described later to prepare a three-point calibration curve, and the concentrations of each target substance in the sample were determined. As the internal standard solution, a 0.1 vol% methyl salicylate/acetone solution was used.

### (GC/MS Measurement Conditions)

Machine: GC HP6890/MS HP5973 (Agilent Technologies, Inc.)
Ionization method: EI (Electron Ionization) method Column: DB-WAX 60 m × 250 µm × 0.5 um (Agilent Technologies, Inc.)
Temperature rise conditions: 70°C (5 min) → 200°C (5 min) Rate = 10°C/min
Inlet temperature: 220°C
AUX temperature: 230°C
Ion source temperature: 230°C
Split ratio: 10:1
Flow rate: 2.0 mL/min
Average linear velocity: 37 cm/sec
Input amount: 1 µL
Measurement mode: SIM

### <Method of Evaluating Heat Resistance>

As an index of heat resistance of the methacrylic resin composition obtained in Examples and Comparative Examples, the heat distortion temperatures (hereinafter referred to as "HDT") (°C) of the test pieces (127 mm of length × 12.7 mm of width × 3 mm of thickness) of the resin molded bodies obtained in Examples and Comparative Examples were measured in accordance with JIS K 7191.

### <Light Stability (ΔYI)>

A UV exposure test was performed using METAL WEATHER ultra-accelerating light stability tester (model name: KU-R5CI-A, manufactured by Daipla Wintes Co., Ltd.) equipped with a metal-halide lamp (model: MW-60W, manufactured by Daipla Wintes Co., Ltd.) and a light-cutting filter (model: KF-1, manufactured by Daipla Wintes Co., Ltd.), and the change in the yellowness index (ΔYI) obtained between before and 200 hours after the start of the UV exposure test was measured according to the method described later.

Specifically, in the evaluation chamber of the METAL WEATHER ultra-accelerating light stability tester, test pieces composed of the methacrylic resin compositions obtained in Examples and Comparative Examples (square shape with 50 mm of length × 50 mm of width, and thickness of 5 mm) were placed.

The radiation intensity of ultraviolet light (UV) irradiated from the metal-halide lamp to the test pieces was corrected such that the radiation intensity measured with a UV irradiance meter (model name: UIT-101, manufactured by Ushio Inc.) at a wavelength of 330 to 390 nm was 80 mW/cm². The test pieces were irradiated with ultraviolet light (radiation intensity: 80 mW/cm²) from the metal-halide lamp, with the evaluation chamber of the METAL WEATHER ultra-accelerating light stability tester set such that it was in an environment at a temperature of 63°C and a humidity of 50% (RH).

As an index of light stability, the yellowness index (YI) of each test piece was measured in accordance with ASTM D 1925 using a spectrophotometric type color difference meter (model name: SE-7700, manufactured by Nippon Denshoku Industries Co., Ltd.). One measurement was performed for each test piece using one test piece obtained before the start of the UV exposure test and one test piece obtained 200 hours after the start, and the change in the measurement was considered as the change in yellowness index (ΔYI).

### <Production of Methacrylic Resin Composition>

### [Example 1]

### (1) Production of Syrup

To a reactor (polymerization vessel) equipped with a condenser, a thermometer, and a stirrer, methyl 2-methylbutyrate was added at a concentration of 300 ppm. In addition, 98.0 parts of MMA and 2.0 parts of BA were supplied. After bubbling with nitrogen gas while stirring, heating was started. When the internal temperature of the reactor reached 80°C, 0.12 parts of 2,2'-azobis-(2,4-dimethylvaleronitrile) as a radical polymerization initiator and 0.075 parts of 1-dodecanethiol as a chain transfer agent were added, followed by further heating until the internal temperature of the reactor reached 100°C, and then the temperature was held for 9 minutes. Then, cooling was performed until the internal temperature of the reactor reached the room temperature to obtain a syrup. The polymer content in the syrup relative to the total mass of the syrup was 25% by mass.

### (2) Casting Polymerization

0.15 parts of t-hexyl peroxypivalate as a radical polymerization initiator was added to 100 parts of the syrup to obtain a polymerizable composition (X2). Next, the polymerizable composition (X2) was cast into a space with a void spacing of 6.5 mm, which was formed by placing soft resin gaskets between edges of two SUS plates facing one another. The polymerizable composition (X2) was heated and cured at 80°C for 30 minutes, then at 130°C for 30 minutes to obtain a methacrylic resin composition. The composition of the methacrylic resin composition is shown in Table 1. Next, the (meth)acrylate resin composition was cooled together with the SUS plates, and the SUS plates were removed to obtain a plate resin molded body with a thickness of 5 mm. The results of evaluation of the characteristics of the obtained resin molded body are shown in Table 1. In Table 1, "-" means that no measurement was performed.

### [Example 2]

### (1) Production of Syrup

To a reactor (polymerization vessel) equipped with a condenser, a thermometer, and a stirrer, methyl 2-methylbutyrate was added at a concentration of 300 ppm. In addition, 100 parts of MMA were supplied. After bubbling with nitrogen gas while stirring, heating was started. When the internal temperature of the reactor reached 80°C, 0.12 parts of 2,2'-azobis-(2,4-dimethylvaleronitrile) as a radical polymerization initiator was added, followed by further heating until the internal temperature of the reactor reached 100°C, and then the temperature was held for 9 minutes. Then, cooling was performed until the internal temperature of the reactor reached the room temperature to obtain a syrup. The polymer content in the syrup relative to the total mass of the syrup was 20% by mass.

### (2) Casting Polymerization

0.15 parts of t-hexyl peroxypivalate as a radical polymerization initiator was added to 100 parts of the syrup to obtain a polymerizable composition (X2). Next, the polymerizable composition (X2) was cast into a space with a void spacing of 4.1 mm, which was formed by placing soft resin gaskets between edges of two SUS plates facing one another. The polymerizable composition (X2) was heated and cured at 80°C for 30 minutes, then at 130°C for 30 minutes to obtain a methacrylic resin composition. The composition of the methacrylic resin composition is shown in Table 1. Next, the methacrylic resin composition was cooled together with the SUS plates, and the SUS plates were removed to obtain a plate resin molded body with a thickness of 3 mm. The results of evaluation of the characteristics of the obtained resin molded body are shown in Table 1. In Table 1, "-" means that no measurement was performed.

### [Examples 3 to 6]

Methacrylic resin compositions and resin molded bodies were produced in the same manner as in Example 2, except that the composition of the monomer composition was changed as described in Table 1. The compositions of the obtained methacrylic resin compositions are shown in Table 1. The results of evaluation of the characteristics of the obtained resin molded bodies are shown in Table 1.

### [Comparative Examples 1 and 2]

Methacrylic resin compositions and resin molded bodies were obtained in the same manner as in Example 1, except that the composition of the monomer composition was changed as described in Table 1. The compositions of the obtained methacrylic resin compositions are shown in Table 1. The results of evaluation of the characteristics of the obtained resin molded bodies are shown in Table 1.

### [Comparative Example 3]

A methacrylic resin composition and a resin molded body were obtained in the same manner as in Example 2, except that the composition of the monomer composition was changed as described in Table 1. The composition of the obtained methacrylic resin composition is shown in Table 1. The results of evaluation of the characteristics of the obtained resin molded body are shown in Table 1.

**Table 1**

| Type | | | | Unit | Ex. 1 | Com. Ex. 1 | Com. Ex. 2 | Ex. 2 | Ex 3 | Ex. 4 | Ex. 5 | Ex. 6 | Com. Ex. 3 |
|---|---|---|---|---|---|---|---|---|---|---|---|---|---|
| Monomer composition | Methylmethacrylate | | MMA | pat by mass | 98.0 | 98.0 | 98.0 | 100.0 | 100.0 | 100.0 | 100.0 | 100.0 | 100.0 |
| | Acryticester | | BA | part by mass | 2.0 | 2.0 | 2.0 | - | - | - | - | - | - |
| | Methyl pyruvate | | | ppm by mass (*1) | - | - | - | - | 300 | 2000 | 10000 | - | - |
| | Methyl 2-methybutyrate | | | ppm by mass (*1) | 300 | - | - | 300 | - | - | - | 4000 | - |
| | Methyl isobutyrate | | | ppm by mass (*1) | - | - | - | - | - | - | - | 8000 | - |
| | Methyl propionate | | | ppm by mass ("1) | - | - | - | - | - | - | - | 4000 - | |
| | Hindered amine compound | | LA-57 | ppm by mass ("1) | - | - | 300 | - | - | - | - | - | - |
| Resin compose | (Meth)acrylic polymer (P) | Methyl methacrylate | MA | % by mass | 97.97 | 98.00 | 98.00 | 99.97 | 99.97 | 99.80 | 99.01 | 98.43 | 100.00 |
| | | Acrylic ester | BA | % by mass | 200 | 200 | 200 | - | - | - | - | - | - |
| | Methyl pyruvate content in resin composition | | | ppm by mass | - | - | . | - | 200 | 1840 | 9200 | - | . |
| | Methyl 2-methylbultyrate content in resin composition | | | ppm by mass (*2) | 290 | - | - | 200 | - | - | - | 3520 | - |
| | Methyl isobutyrate content in resin composition | | | ppm by mass (*2) | - | - | - | - | - | - | - | 7700 | - |
| | Methyl propionate content in resin composition | | | ppm by mass (*2) | - | - | - | - | - | - | - | 3900 | - |
| Evaluation result of resin molded body | YI | | | 0 h | 0.58 | 0.44 | 0.43 | 0.23 | 0.33 | 0.38 | 0.33 | 0.25 | 0.28 |
| | | | | 200 h | 3.00 | 4.08 | 3.00 | 5.92 | 7.07 | 4.41 | 0.90 | 3.10 | 1231 |
| | light stability (ΔYI) | | | - | 3.02 | 3.64 | 3.42 | 5.69 | 6 74 | 403 | 0.57 | 2.85 | 12.03 |
| | Heat resistance | | | 103.00 | | 102.30 | 102.30 | 102.90 | 103.20 | 103.10 | 101.20 | 99.40 | 103.00 |

| | | | | | | | | | | | | | |
|---|---|---|---|---|---|---|---|---|---|---|---|---|---|
| *1 Content relative to the total mass of methyl methacrylate (MMA) *2 Content relative to the total mess of resin composition (resin molded body) | | | | | | | | | | | | | |

Comparison between Example 1 and Comparative Example 1 demonstrates that at least one compound of methyl pyruvate and methyl 2-methylbutyrate can be contained in a system in which methacrylic polymer contains an acrylic ester-derived unit to improve the light stability.

Comparison between Example 1 and Comparative Example 2 demonstrates that the cases where the methacrylic polymer contains at least one compound of methyl pyruvate and methyl 2-methylbutyrate, even if the resin composition does not contain a hindered amine compound, can achieve better light stability than the cases where a hindered amine compound is contained.

Comparison among Examples 2 to 6 and Comparative Example 3 demonstrates that at least one compound of methyl pyruvate and methyl 2-methylbutyrate can be contained in a system in which methacrylic polymer does not contain an acrylic ester-derived unit to improve the light stability.

## Claims

1. A monomer composition comprising methyl methacrylate, and at least one compound of methyl pyruvate and methyl 2-methylbutyrate.

2. A monomer composition comprising methyl methacrylate, and at least one compound of methyl pyruvate and methyl 2-methylbutyrate,
wherein the total content of the at least one compound of methyl pyruvate and methyl 2-methylbutyrate is 5 ppm by mass or more relative to the total mass of the monomer composition.

3. A monomer composition comprising methyl methacrylate, and at least one compound of methyl pyruvate and methyl 2-methylbutyrate,
wherein the total content of the at least one compound of methyl pyruvate and methyl 2-methylbutyrate is 50 ppm by mass or more relative to the total mass of the monomer composition.

4. A monomer composition comprising methyl methacrylate, and at least one compound of methyl pyruvate and methyl 2-methylbutyrate,
wherein the total content of the at least one compound of methyl pyruvate and methyl 2-methylbutyrate is 100 ppm by mass or more relative to the total mass of the monomer composition.

5. The monomer composition according to any one of claims 1 to 4, further comprising an acrylic ester.

6. The monomer composition according to claim 5, wherein the acrylic ester is at least one compound selected from the group consisting of methyl acrylate, n-ethyl acrylate, and n-butyl acrylate.

7. The monomer composition according to claim 5, wherein the acrylic ester is n-butyl acrylate.

8. The monomer composition according to any one of claims 1 to 7, further comprising at least one compound of methyl isobutyrate and methyl propionate.

9. A methacrylic resin composition comprising a radical polymerization product of a polymerizable composition (X2) comprising the monomer composition according to any one of claims 1 to 8.

10. A methacrylic resin composition comprising methacrylic polymer (P), and at least one compound of methyl pyruvate and methyl 2-methylbutyrate.

11. A methacrylic resin composition comprising methacrylic polymer (P), and at least one compound of methyl pyruvate and methyl 2-methylbutyrate,
wherein the total content of the at least one compound of methyl pyruvate and methyl 2-methylbutyrate is 5 ppm by mass or more relative to the total mass of the methacrylic resin composition.

12. A methacrylic resin composition comprising methacrylic polymer (P), and at least one compound of methyl pyruvate and methyl 2-methylbutyrate,
wherein the total content of the at least one compound of methyl pyruvate and methyl 2-methylbutyrate is 50 ppm by mass or more relative to the total mass of the methacrylic resin composition.

13. A methacrylic resin composition comprising methacrylic polymer (P), and at least one compound of methyl pyruvate and methyl 2-methylbutyrate,
wherein the total content of the at least one compound of methyl pyruvate and methyl 2-methylbutyrate is 100 ppm by mass or more relative to the total mass of the methacrylic resin composition.

14. The methacrylic resin composition according to any one of claims 10 to 13, wherein the methacrylic polymer (P) comprises 70 to 100% by mass of a repeating unit derived from methyl methacrylate, and 0 to 30% by mass of a repeating unit derived from an acrylic ester.

15. The methacrylic resin composition according to any one of claims 10 to 13, wherein the methacrylic polymer (P) comprises 50 to 100% by mass of a repeating unit derived from methyl methacrylate, and 0 to 50% by mass of a repeating unit derived from styrene.

16. A resin molded body comprising the methacrylic resin composition according to any one of claims 9 to 15.

17. A method of producing a methacrylic resin composition, comprising a radical polymerization step of subjecting a polymerizable composition (X2) comprising the monomer composition according to any one of claims 1 to 8 to radical polymerization.
